# FASCICULE DE BREVET EUROPEEN

(11) **EP 4 009 145 B1**
(45) Date de publication et mention de la délivrance du brevet: **10.01.2024**
(21) Numéro de dépôt: 21210952.4
(22) Date de dépôt: 29.11.2021
(51) Int. Cl.: G06F 3/01

(54) **DISPOSITIF HAPTIQUE MULTIMODAL**
MULTIMODALE HAPTISCHE VORRICHTUNG
MULTI-MODAL HAPTIC DEVICE

(30) Priorité: 01.12.2020 FR 2012475
(43) Date de publication de la demande: 08.06.2022
(73) Titulaire: Commissariat à l'Energie Atomique et aux Energies Alternatives, 75015 Paris (FR)
(72) Inventeur: OLLIER, Emmanuel, 38054 GRENOBLE Cedex 09 (FR); CAROFF, Tristan, 38054 GRENOBLE Cedex 09 (FR); CASSET, Fabrice, 38054 GRENOBLE Cedex 09 (FR)
(74) Mandataire: Atout PI Laplace

(56) Documents cités:
- WO-A1-2018/143418
- KR-A- 20120 018 245
- KR-B1- 101 767 485
- US-A1- 2015 220 199
- US-A1- 2019 278 374
- SINGHAL ANSHUL ET AL: "Perceptual interactions in thermo-tactile displays", 2017 IEEE WORLD HAPTICS CONFERENCE (WHC), IEEE, 6 juin 2017 (2017-06-06), pages 90-95, XP033131395, DOI: 10.1109/WHC.2017.7989882 [extrait le 2017-07-21]
- ABAD ALEXANDER C ET AL: "An Untethered Multimodal Haptic Hand Wearable", 2021 IEEE SENSORS, IEEE, 31 octobre 2021 (2021-10-31), pages 1-4, XP034052082, DOI: 10.1109/SENSORS47087.2021.9639526 [extrait le 2021-12-06]

## Description

### DOMAINE TECHNIQUE DE L'INVENTION

L'invention se situe dans le domaine de l'haptique, et concerne plus particulièrement un dispositif haptique multimodal, c'est-à-dire un dispositif apte à appliquer à un utilisateur des forces, des pressions, des vibrations et/ou des mouvements ainsi que des différences de température pour lui transmettre différentes sensations ou différentes informations (texture, viscosité, forme, force, chaleur...). Plus précisément, le dispositif haptique se présente sous la forme d'une matrice haptique multimodale comprenant une ou plusieurs cellules intégrées dans la matrice.

### ETAT DE LA TECHNIQUE

Le terme haptique désigne la science du toucher. A la différence du terme tactile qui ne définit que le sens du toucher, l'haptique englobe la dimension kinesthésique du toucher, c'est-à-dire la perception du corps dans l'environnement.

Un dispositif haptique est un système tactile et kinesthésique qui peut notamment créer une communication entre un humain et une partie de son environnement, le cas échéant entre un utilisateur et un environnement virtuel. Un dispositif haptique peut par exemple permettre à un utilisateur de concevoir, modeler et manipuler des objets dans un environnement virtuel avec un ressenti tactile (toucher) et une perception kinesthésique.

Plusieurs technologies combinent un retour tactile et kinesthésique, souvent en montant un écran tactile sur un dispositif de retour de force. Ces technologies mettent généralement en oeuvre des moyens essentiellement mécaniques avec par exemple des moteurs miniaturisés ou des éléments piézoélectriques générant une vibration qui peut certes être modulée, mais ne permet généralement pas de fournir les informations tactiles réalistes. Le retour haptique est ainsi généralement insuffisant pour rendre tangible l'environnement, et manque considérablement de réalisme. La perception humaine du toucher est en effet multimodale, l'être humain ayant besoin de plusieurs informations, tels que la forme, la texture, la température pour identifier un objet au toucher. Fait intéressant, même en l'absence d'autres informations tactiles, l'être humain est en mesure de distinguer une variété de matériaux en se basant uniquement sur les signaux thermiques.

Certaines technologies intègrent des éléments piézoélectriques dans une matrice souple tel qu'un polymère silicone souple afin de mieux interagir avec le corps humain. D'autres technologies à base d'éléments microfluidiques permettent de générer des pressions locales sur une main et donc une certaine sensation de toucher. Cependant, il n'en reste pas moins que ces technologies ne transmettent qu'une quantité d'informations insuffisante pour permettre une perception réaliste du toucher humain.

D'autres technologies intègrent des éléments pour générer des effets haptiques thermiques.

Le brevet US 9 703 381 décrit un dispositif haptique multimodal comprenant un premier récipient et un deuxième récipient, chacun contenant un fluide à une température spécifique (par exemple un fluide chaud et un fluide froid), une première canalisation et une deuxième canalisation, chacune connectée par un collecteur au premier conteneur et au deuxième conteneur, et au moins une unité d'affichage tactile connectée aux première et deuxième canalisations, unité qui est en fait une unité de transmission tactile apte à transmettre des informations tactiles à un corps. Le collecteur comprend une pompe et une vanne pour contrôler et réguler la circulation des fluides dans les canalisations. Les récipients sont couplés ensemble par un élément Peltier qui peut chauffer le premier récipient alors qu'il refroidit le second récipient. Ainsi, le fluide chaud circule dans la première canalisation, le fluide froid circule dans la seconde canalisation, et les fluides sont mélangés selon des proportions qui peuvent être réglées avant d'atteindre l'unité de transmission tactile pour transmettre une sensation de chaleur qui peut ainsi être modulée. L'unité de transmission tactile peut comprendre une chambre gonflable faite d'un matériau élastique et peut transmettre un retour tactile. Le dispositif décrit est donc basé sur des éléments fluidiques, l'élément Peltier permettant de moduler la chaleur de ces éléments, intervenant donc indirectement dans la transmission de chaleur au corps. Le dispositif décrit est donc relativement complexe, avec de nombreux éléments, ce qui en fait un dispositif relativement volumineux, avec des risques de fuite de fluide, et potentiellement des pertes thermiques.

Un autre dispositif haptique multimode est présenté dans la publication « Altered Touch: Miniature Haptic Display with Force, Thermal and Tactile Feedback for Augmented Haptics », Takaki Murakami, Tanner Person, Charith Lasantha Fernando, and Kouta Minamizawa (SIGGRAPH 2017 Emerging Technologies, July 2017 Article No.: 2 Pages 1-2, DOI: http://dx.doi.org/10.1145/3084822.3084836). Le dispositif haptique multimode décrit comprend un module Peltier associé à des moteurs pour générer les effets tactiles et thermiques. Néanmoins, il s'agit d'un module uniquement adapté pour le bout des doigts. Il est relativement volumineux. Il ne peut intégrer qu'un module Peltier, qui est impossible à répéter pour obtenir une grande surface de perception,

Le document US 2015/220199 A1 décrit un dispositif haptique multimodal comprenant une matrice intégrant au moins une cellule, chaque cellule comportant : au moins un élément thermique apte à générer un refroidissement et un chauffage ; au moins un élément vibratoire apte à générer une vibration ; et au moins une couche d'encapsulation en un matériau électriquement isolant. Le au moins un élément vibratoire et le au moins un élément thermique étant ancrés en au moins une partie dans ladite au moins une couche d'encapsulation ; chaque cellule étant adaptée pour être en contact directement ou indirectement avec la peau d'une personne de manière à lui transmettre des sensations thermique et/ou vibratoire, ladite cellule présentant une épaisseur inférieure ou égale à dix millimètres.

Le document KR 101 767 485 B1 décrit un autre dispositif haptique multimodal comportant au moins un élément thermique apte à générer un chauffage, et au moins un élément vibratoire apte à générer une vibration.

D'autres dispositifs haptiques sont décrits dans les documents WO 2018/143418 A1, US 2019/278374 A1, KR 2012 0018245 A, SINGHAL ANSHUL ET AL: "Perceptual interactions in thermo-tactile displays", 2017 IEEE WORLD HAPTICS CONFERENCE (WHC), IEEE, 6 juin 2017, et ABAD ALEXANDER C ET AL: "An Untethered Multimodal Haptic Hand Wearable", 2021 IEEE SENSORS, IEEE, 31 octobre 2021.

D'autres technologies s'intéressent à la fabrication de modules Peltier flexibles sous forme de couches minces flexibles (plutôt que les modules Peltier à « jambes » comme ceux décrits précédemment et généralement utilisés) qui peut permettre de réduire le volume d'un module Peltier. Une technologie est décrite par exemple dans la publication « High-performance and compact-designed flexible thermoelectric modules enabled by a reticulate carbon nanotube architecture » (Nature Communications, DOI: 10.1038/ncomms14886) qui présente des modules Peltier réalisés à partir de dépôts épais de nanotubes de carbone dopés par une solution de polyethyleneimine (PEI) dans l'éthanol. Ce procédé permet d'obtenir des dopages p et n et de réaliser des modules flexibles. Cependant, un tel module Peltier permet des variations de températures dans le plan de la couche mince et non perpendiculairement à ladite couche. En outre, cela ne forme pas dispositif haptique multimodal.

L'invention vise à surmonter les inconvénients précités de l'art antérieur.

Plus particulièrement, l'invention vise à disposer d'un dispositif haptique multimodal permettant de transmettre non seulement des sollicitations mécaniques ou vibratoires (désigné par « mode vibratoire » par la suite) mais également des sollicitations en température (désigné par « mode thermique » par la suite) à un corps, et qui soit d'épaisseur réduite, c'est à dire de quelques millimètres, voire moins d'un millimètre d'épaisseur.

De préférence, il est recherché un dispositif haptique multimodal flexible, pouvant s'adapter à différentes morphologies, voire pouvant constituer une sorte de seconde peau.

Il est en outre recherché un dispositif haptique multimodal pouvant être piloté à la fois et simultanément selon les modes vibratoire et thermique.

### EXPOSE DE L'INVENTION

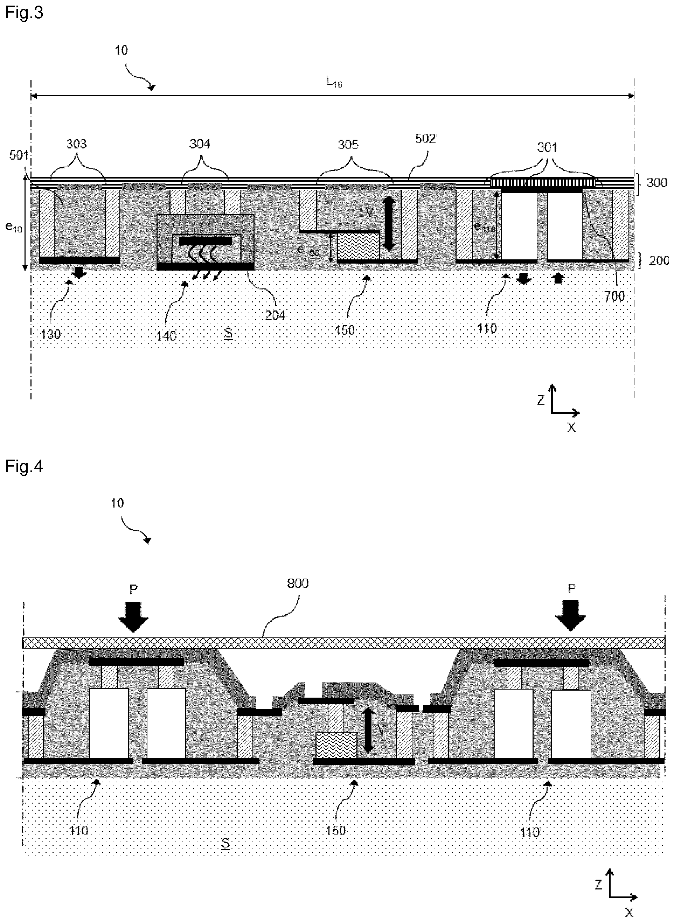

L'objet de l'invention permettant de remédier à ces inconvénients est un dispositif haptique multimodal, ledit dispositif comprenant une matrice intégrant au moins une cellule, chaque cellule comportant :
- au moins un élément thermique apte à générer un refroidissement et un chauffage ;
- au moins un élément vibratoire apte à générer une vibration ; et
- au moins une couche d'encapsulation en un matériau électriquement isolant ;
   le au moins un élément vibratoire et le au moins un élément thermique étant ancrés en au moins une partie dans ladite au moins une couche d'encapsulation ;
   chaque cellule étant adaptée pour être en contact directement ou indirectement avec la peau d'une personne de manière à lui transmettre des sensations thermique et/ou vibratoire, ladite cellule présentant une épaisseur inférieure ou égale à dix millimètres, le au moins un élément thermique présentant une hauteur supérieure à celle du au moins un élément vibratoire,
   au moins une cellule comprenant:
      - un élément vibratoire central et ;
      - au moins deux modules Peltier disposés autour dudit élément vibratoire et formant ledit au moins un élément thermique.
L'invention telle que revendiquée est décrite dans le contexte de la Figure 4. Les autres réalisation décrites dans la description ne citent pas nécessairement la combinaison complète des caractéristiques telle que revendiquée, mais permettent de mieux comprendre le contexte de l'invention.

Par couche d'encapsulation, on entend une couche qui est conformée pour ancrer au moins une partie des éléments thermique(s) et vibratoire(s) d'une cellule et/ou d'une matrice. Une couche d'encapsulation n'entoure pas nécessairement entièrement les éléments thermique(s) et vibratoire(s).

Selon l'invention, les termes « épaisseur » et « hauteur » désignent la dimension selon la direction perpendiculaire à la peau d'une personne lorsque le dispositif haptique est disposé sur ladite peau, directement ou indirectement. La direction longitudinale désigne la direction correspondant à la plus grande dimension d'une cellule, d'une matrice ou d'un dispositif dans son plan principal. Les termes « en-dessous », « au-dessus » sont à comprendre par référence à la direction perpendiculaire.

Une cellule peut présenter une longueur entre 1 et 20 millimètres et une largeur comprise entre 1 et 20 millimètres.

Le dispositif haptique selon l'invention est donc un dispositif haptique multimodal permettant de transmettre non seulement des sollicitations vibratoires mais également des sollicitations thermiques à la peau d'une personne, et sa structure sous forme de matrice à une ou plusieurs cellules lui confère une épaisseur réduite.

Le dispositif selon l'invention peut en outre comporter l'une ou plusieurs des caractéristiques suivantes prises isolément ou suivant toutes les combinaisons techniques possibles.

De préférence, au moins une cellule comprend en outre :
- une pluralité de pistes conductrices inférieures, aptes à réaliser les connexions électriques au niveau des parties inférieures des éléments thermique et vibratoire ; et
- une pluralité de pistes conductrices supérieures, aptes à réaliser les connexions électriques au niveau des parties supérieures des éléments thermique et vibratoire, et de préférence au moins le routage pour le pilotage desdits éléments.

Les pistes conductrices inférieures et/ou supérieures peuvent être en métal (par exemple en cuivre, or, aluminium, ou nickel), ou en carbone, ou encore à base de particules conductrices mises en oeuvre par exemple sous forme d'encres conductrices (par exemple à base de PEDOT-PSS, acronyme de poly(3,4-éthylènedioxythiophène) (PEDOT) et de polystyrène sulfonate) de sodium (PSS)).

De préférence, la au moins une cellule comprend une pluralité de vias conducteurs, lesdits vias permettant de réaliser des connexions électriques verticales entre les pistes conductrices supérieures et les éléments thermique et vibratoire.

Les vias peuvent être en cuivre, or, aluminium et/ou être des colles conductrices.

Selon un mode de réalisation, la au moins une couche d'encapsulation comprend une première couche d'encapsulation en un premier matériau isolant et une deuxième couche d'encapsulation en un deuxième matériau isolant, les première et deuxième couches pouvant être jointes, et/ou les premier et deuxième matériaux isolants pouvant être identiques.

Selon un mode de réalisation particulier, la deuxième couche d'encapsulation intègre les pistes conductrices supérieures, formant une couche de redistribution des interconnexions, ladite couche de redistribution étant de préférence flexible.

Selon un mode de réalisation préféré, au moins un élément thermique est un élément Peltier, de préférence au moins deux éléments Peltier de type N et P connectés électriquement en série pour former un module Peltier.

En particulier, au moins une cellule peut comprendre un moyen de dissipation thermique associé à au moins un module Peltier. Cela permet de garantir un fonctionnement optimal en mode refroidissement d'un module Peltier.

Selon un mode de réalisation, pouvant être combiné avec les modes précédents, au moins un élément thermique est une résistance chauffante et/ou un élément radiant, par exemple un élément radiant Infrarouge (IR). Une résistance chauffante peut être une résistance en graphite (C) ou une résistance en argent (Ag).

Un élément vibratoire peut être un élément piézoélectrique, un élément ferroélectrique et/ou un élément électromagnétique.

Selon un mode de réalisation particulier, au moins un élément thermique et/ou vibratoire, de préférence tous les éléments thermique et/ou vibratoire, sont entièrement encapsulé(s) dans au moins une couche d'encapsulation.

La (ou les) couche(s) d'encapsulation peu(ven)t être en un ou plusieurs des matériaux suivants : un polymère tels qu'un polytéréphtalate d'éthylène (PET), un polynaphtalate d'éthylène (PEN), un polyimide (PI), un polycarbonate (PC) ou un silicone ; un verre ; un métal ; ou encore un matériau non organique tel que l'oxyde de silicium.

La (ou les) couche(s) d'encapsulation peu(ven)t être en un matériau flexible et/ou étirable, par exemple un polymère flexible et/ou un élastomère.

Selon un mode de réalisation, pouvant être combiné avec les modes précédents, au moins une cellule comprend en outre au moins une piste chauffante complémentaire sous au moins un élément thermique et/ou vibratoire, et de préférence sous au moins une piste conductrice inférieure.

Selon un mode de réalisation, pouvant être combiné avec les modes précédents, au moins une cellule comporte des amincissements entre au moins deux éléments thermique et/ou vibratoire dans l'épaisseur d'au moins une couche d'encapsulation. Cela permet de rendre la cellule plus flexible.

Selon un mode de réalisation, pouvant être combiné avec les modes précédents, au moins une cellule comporte un élément de pression, notamment une couche de polymère ou de plastique, éventuellement élastique, disposée sur l'ensemble de ladite cellule en prenant appui sur au moins un élément, apte à exercer indirectement une pression contre la peau. Ceci permet de favoriser un meilleur contact thermique de la cellule avec la peau et de favoriser le comportement vibratoire de la membrane formée par le matériau encapsulant actionnée par l'élément vibratoire.

Selon l'invention, les modules Peltier peuvent présenter des hauteurs supérieures à la hauteur de l'élément vibratoire. Les modules Peltier avec le matériau d'encapsulation peuvent ainsi former un raidisseur pour l'actionnement de l'élément vibratoire, améliorant ainsi le fonctionnement vibratoire.

La matrice comprend de préférence plusieurs cellules, encore plus préférentiellement plusieurs cellules identiques.

Selon un mode de réalisation, au moins deux éléments thermique et/ou vibratoire sont disposés l'un au-dessus de l'autre dans une même cellule et/ou une matrice de cellules.

Selon un mode de réalisation alternatif ou complémentaire au mode précédent, au moins deux éléments thermique et/ou vibratoire sont disposés l'un à côté de l'autre dans une même cellule et/ou une matrice de cellules.

### BREVE DESCRIPTION DES FIGURES

D'autres caractéristiques, détails et avantages de l'invention ressortiront à la lecture de la description faite en référence aux dessins annexés donnés à titre d'exemple et qui représentent, respectivement :
La Fig.1 représente un premier mode de réalisation d'un dispositif haptique multimodal
La Fig.2 représente un deuxième mode de réalisation d'un dispositif haptique multimodal
La Fig.3 représente un troisième mode de réalisation d'un dispositif haptique multimodal
La Fig.4 représente un quatrième mode de réalisation d'un dispositif haptique multimodal conforme à l'invention.
La Fig.5 représente un cinquième mode de réalisation d'un dispositif haptique multimodal
Les Fig.6A, Fig.6B, Fig.6C et Fig.6D représentent plusieurs exemples de configurations entre un élément vibratoire et des éléments thermiques dans le plan d'un dispositif haptique multimodal
Les Fig.7A, Fig.7B et Fig.7C représentent différents moyens de dissipation thermique associés à un module Peltier d'un dispositif haptique multimodal
Les Fig.8A, Fig.8B, Fig.8C et Fig.8D représentent quatre variantes correspondant à la configuration de la figure 6A.
Les Fig.9A et Fig.9B représentent un sixième mode de réalisation, selon deux variantes, d'un dispositif haptique multimodal
La Fig.10 représente une matrice constituée de plusieurs cellules d'un dispositif haptique multimodal
La Fig.11 représente un exemple de procédé de réalisation d'un dispositif haptique multimodal

Dans l'ensemble de ces figures, des références identiques peuvent désigner des éléments identiques ou analogues.

De plus, les différentes parties représentées sur les figures ne le sont pas nécessairement selon une échelle uniforme, pour rendre les figures plus lisibles.

### DESCRIPTION DETAILLEE DE L'INVENTION

Dans l'ensemble des modes de réalisation, une cellule 10 s'étend dans un plan principal XY. Elle présente une épaisseur inférieure ou égale à 10 millimètres, de préférence comprise entre 0,1 et 5 millimètres. Par exemple, elle peut présenter une épaisseur comprise entre 1 et 5 mm dans le cas d'une intégration verticale et entre 0,1 et 1 mm dans le cas d'une intégration dans le plan.

Une cellule 10 peut présenter une longueur (dimension dans la direction X) entre 1 et 20 millimètres et une largeur (dimension dans la direction Y) comprise entre 1 et 20 millimètres.

Le matériau (ou les matériaux d'encapsulation) peu(ven)t être flexible(s) et/ou étirable(s), mais il(s) peu(ven)t également être rigide(s) pour être disposé(s) par exemple sur une interface de type Smartphone.

Bien que les éléments vibratoires soient généralement présentés sous la forme d'éléments piézoélectriques, ceci n'est pas limitatif, et ils peuvent être également des éléments ferroélectriques et/ou électromagnétiques.

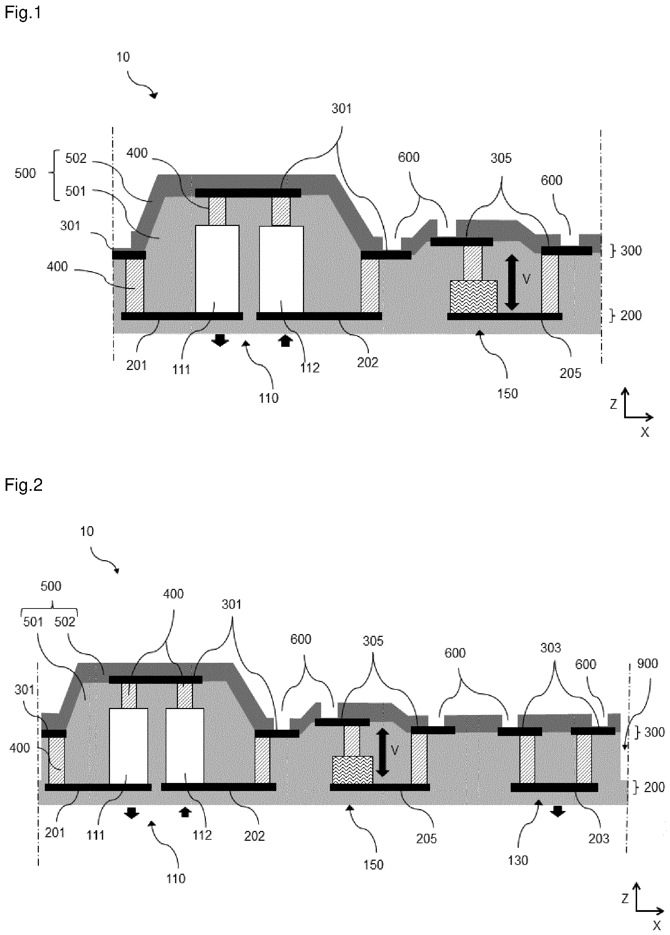

Dans les figures 1 à 8D, les différents éléments d'une cellule sont disposés les uns à côté des autres dans le plan XY, et notamment dans une direction X du plan XY. Cette intégration des différents éléments dans le plan est préférée car elle permet d'obtenir l'épaisseur la plus faible, permettant notamment d'augmenter la flexibilité/souplesse d'une cellule. En outre, elle permet de favoriser la dynamique et l'efficacité des transmissions grâce à la proximité immédiate des éléments avec la peau, autant pour chauffer et refroidir la surface de la peau que pour lui transmettre les vibrations. Les faibles dimensions des différents éléments permettent en outre de conserver une résolution spatiale proche de la résolution naturelle obtenue avec les récepteurs mécaniques et thermiques de la peau, ce qui permet de reproduire une sensation localisée sur la peau.

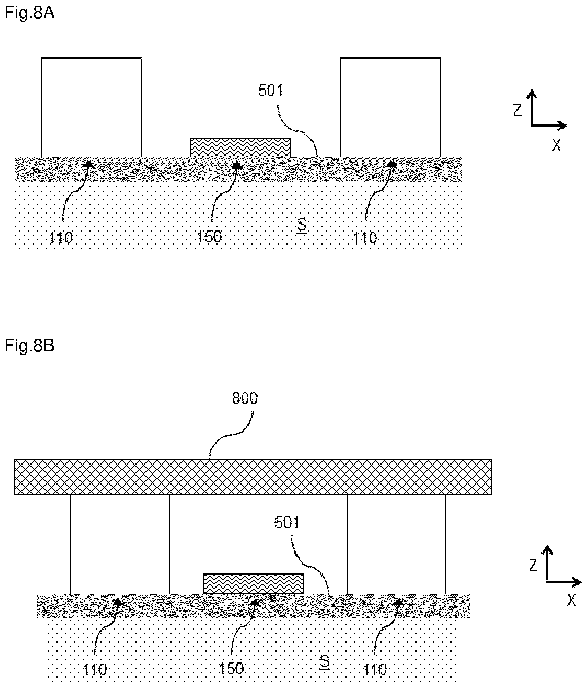

En outre, dans les figures 1 à 7C, 9A et 9B, la au moins une couche d'encapsulation est conformée pour encapsuler entièrement les éléments thermique et vibratoire. Cependant, ceci n'est pas limitatif. Comme illustré dans les figures 8A à 8D, la au moins une couche d'encapsulation est conformée pour ancrer seulement une partie des éléments thermique et vibratoires d'une cellule.

La figure 1 représente un premier mode de réalisation d'un dispositif haptique multimodal conforme à l'invention, plus précisément d'une cellule comprise dans un dispositif haptique multimodal conforme à l'invention.

La cellule 10 comporte :
- un module Peltier 110 comprenant deux éléments Peltier 111, 112, chaque module étant apte à générer un refroidissement et/ou un échauffement selon la polarisation électrique appliquée audit élément ;
- un élément vibratoire 150 disposé à côté du module Peltier dans la direction X ; et
- au moins une couche d'encapsulation 500 en un matériau d'encapsulation isolant électriquement.

On a représenté un module Peltier à deux éléments, mais généralement un module Peltier comporte plus de deux éléments, ceci étant applicable pour tous les modes de réalisation et plus généralement pour une cellule ou une matrice conforme à l'invention.

Typiquement, un module Peltier comporte des éléments Peltier dopés P connectés alternativement avec des éléments de type N, cette disposition permet d'absorber la chaleur sur une face supérieure, respectivement inférieure, du module et rejeter la chaleur sur l'autre face inférieure, respectivement supérieure. Les éléments doivent être connectés électriquement entre eux en série.

Alternativement, on peut faire fonctionner les éléments Peltier du module uniquement en mode refroidissement. Dans ce cas on peut prévoir un autre élément thermique pour le chauffage, telle une résistance chauffante et/ou un élément radiant comme ceci est décrit plus après.

Typiquement, l'élément vibratoire est un élément piézoélectrique.

Une pluralité 200 de pistes conductrices inférieures 201, 202, 205, permet de réaliser les connexions électriques 201, 202 sur les parties inférieures des éléments Peltier 111, 112 et la prise de contact électrique 205 sur la partie inférieure de l'élément vibratoire 150.

Une pluralité 300 de pistes conductrices supérieures 301, 305, permet de réaliser les connexions électriques supérieures entre les éléments Peltier et le routage des signaux de pilotage des éléments Peltier (pistes 301), ainsi que le routage des signaux de pilotage de l'élément vibratoire (pistes 305).

Les pistes conductrices inférieures et/ou supérieures peuvent être en métal (par exemple en cuivre, or, aluminium, ou nickel), ou en carbone, ou encore à base de particules conductrices mises en oeuvre par exemple sous forme d'encres conductrices (par exemple à base de PEDOT-PSS, acronyme de poly(3,4-éthylènedioxythiophène) (PEDOT) et de polystyrène sulfonate) de sodium (PSS)).

Les connexions électriques verticales entre les pistes conductrices supérieures et les éléments sont réalisées par des vias 500 en un matériau conducteur, par exemple en cuivre, or, aluminium et/ou des colles conductrices.

Tous les éléments sont entièrement encapsulés dans au moins une couche d'encapsulation 500, formant ainsi ladite cellule.

Dans la cellule représentée, il y a deux couches d'encapsulation 501, 502 jointes entre elles : une première couche ou couche inférieure 501 (en un premier matériau d'encapsulation) et une deuxième couche ou couche supérieure 502 (en un deuxième matériau d'encapsulation).

La première couche d'encapsulation 501 permet d'encapsuler entièrement les deux éléments Peltier 111, 112 et l'élément vibratoire 150. Elle permet également d'encapsuler les pistes conductrices inférieures 200, les vias 400 et éventuellement les pistes conductrices supérieures 300 jusqu'à une certaine hauteur. La deuxième couche d'encapsulation 502 permet de recouvrir les pistes conductrices supérieures 300.

La deuxième couche d'encapsulation 502 est ouverte localement (ouvertures ou « pads » 600) sur les pistes conductrices supérieures 300 pour permettre les connexions de pilotage de l'ensemble des éléments.

Les premier et deuxième matériaux d'encapsulation peuvent être choisis parmi un ou plusieurs des matériaux suivants : des polymères tels que Polytéréphtalate d'éthylène (PET), Polynaphtalate d'éthylène (PEN), Polyimide (PI), Polycarbonate (PC), Silicone ; un verre fin ; ou encore un métal fin ; voire un matériau non organique tel que l'oxyde de silicium. Ils peuvent être un même matériau ou en plusieurs matériaux différents.

Les matériaux d'encapsulation peuvent être des matériaux flexibles, par exemple des élastomères.

Le premier et/ou le deuxième matériau d'encapsulation est de préférence un matériau diélectrique, notamment pour former l'isolant électrique entre les éléments et les pistes de connexions électriques, en particulier supérieures.

La cellule 10 est destinée à être en contact avec la peau d'une personne, et ce, directement ou indirectement (par exemple par l'intermédiaire d'un tissu).

L'élément vibratoire et les éléments thermiques sont intégrés côte à côte dans le plan principal de la cellule, et ici plus précisément dans la direction longitudinale X de la cellule 10.

Dans ce premier mode de réalisation, la hauteur de l'élément vibratoire est inférieure aux hauteurs des éléments Peltier. Ceci permet d'améliorer les performances des éléments, et notamment de l'élément vibratoire, comme cela sera expliqué plus après. En outre, cette variation de hauteur est répercutée par une variation des épaisseurs des couches d'encapsulation, et notamment de la couche inférieure 501. Une topologie particulière en résulte, qui présente un avantage, comme ceci sera développé plus après également. De manière plus générale, dans l'invention, le ou les éléments thermiques présentent une hauteur supérieure à celle du ou des éléments vibratoires afin d'améliorer les performances des éléments, et notamment de l'élément vibratoire.

La figure 2 représente un deuxième mode de réalisation d'un dispositif haptique multimodal, plus précisément d'une cellule comprise dans un dispositif haptique multimodal. Ladite cellule se distingue de celle du premier mode en ce qu'elle comprend en outre une résistance chauffante 130 qui est formée par une piste conductrice inférieure supplémentaire 203 et qui est disposée à côté de l'élément vibratoire 150 dans la direction X. Deux pistes conductrices supérieures supplémentaires 303 permettent de réaliser la continuité du contact électrique de la résistance chauffante 130 et le routage des signaux de pilotage de ladite résistance.

La résistance chauffante peut être une résistance en graphite (C) ou une résistance en argent (Ag), par exemple une résistance obtenue à partir d'une encre comprenant des particules (éventuellement de taille nanométrique) par exemple de carbone ou d'argent (Nano Ag par exemple).

La première couche d'encapsulation 501 permet d'encapsuler entièrement les deux éléments Peltier 111, 112, la résistance chauffante 130 et l'élément vibratoire 150. Elle permet en outre d'encapsuler les pistes conductrices inférieures 200, les vias 400 et éventuellement les pistes conductrices supérieures 300 jusqu'à une certaine hauteur. La deuxième couche d'encapsulation 502 permet de recouvrir les pistes conductrices supérieures 300.

Comme pour le premier mode de réalisation, l'élément vibratoire et les éléments thermiques (éléments Peltier et résistance chauffante) sont intégrés côte à côte dans le plan principal de la cellule, et ici plus précisément dans la direction longitudinale X de la cellule 10.

Les hauteurs de l'élément vibratoire et de la résistance chauffante sont inférieures aux hauteurs des éléments Peltier. En outre, cette variation de hauteur est répercutée par une variation des hauteurs des deux couches d'encapsulation, et notamment de la couche inférieure 501. Une topologie particulière en résulte, qui présente un avantage, comme ceci sera développé plus après également.

Il est à noter que si le module Peltier est utilisé pour chauffer en plus de sa fonction de refroidissement, alors la résistance chauffante peut ne pas être nécessaire et on peut se retrouver dans le premier mode de réalisation.

La figure 3 représente un troisième mode de réalisation d'un dispositif haptique multimodal, plus précisément d'une cellule comprise dans un dispositif haptique multimodal, représentée posée sur la peau S. Ladite cellule se distingue de celle du deuxième mode en ce qu'elle comprend en outre un élément radiant 140, et en ce que la cellule présente une topologie plate, bien que les hauteurs des différents éléments soient différentes, le module Peltier présentant une hauteur supérieure aux autres éléments.

L'élément radiant peut être un élément radiant Infrarouge (IR), par exemple un élément radiant à basse température pour produire une sensation lente de chaleur ou haute température à l'inverse pour une sensation rapide de chaleur. Un élément radiant peut ainsi être adapté aux chauffages rapides, alors qu'une résistance chauffante est davantage adaptée aux chauffages lents. Ainsi les deux éléments thermiques peuvent être complémentaires.

Une piste conductrice supplémentaire 204 est prévue sous l'élément radiant. Deux pistes conductrices supérieures supplémentaires 304 permettent le routage des signaux de pilotage de l'élément radiant 140.

La première couche d'encapsulation 501 permet d'encapsuler entièrement les deux éléments Peltier 111, 112, la résistance chauffante 130, l'élément radiant 140 et l'élément vibratoire 150. Elle permet en outre d'encapsuler les pistes conductrices inférieures 200, les vias 400 et éventuellement les pistes conductrices supérieures 300 jusqu'à une certaine hauteur. La deuxième couche d'encapsulation 502' permet de recouvrir les pistes conductrices supérieures 300.

En outre, les pistes conductrices supérieures sont représentées intégrées dans une couche de redistribution des interconnexions, formant une couche d'encapsulation supérieure 502' flexible et qui permet d'effectuer le routage de toutes les pistes nécessaires au pilotage des différents éléments des cellules et de rassembler toutes ces pistes en une sortie commune et flexible. Cette caractéristique n'est pas dépendante de l'ajout d'un élément radiant, elle peut être donc appliquée de manière indépendante pour tout autre mode de réalisation, ou plus largement pour un dispositif haptique multimodal.

De plus, la cellule représentée comprend un dissipateur thermique 700 au-dessus du module Peltier. Ce dissipateur thermique est décrit plus après.

Comme pour les premier et deuxième modes de réalisation, l'élément vibratoire et les éléments thermiques (éléments Peltier, résistance chauffante et élément radiant) sont intégrés côte à côte dans le plan principal de la cellule, et ici plus précisément dans la direction longitudinale X de la cellule 10.

La figure 4 représente un quatrième mode de réalisation d'un dispositif haptique multimodal conforme à l'invention, représenté posé sur la peau S, plus précisément d'une cellule comprise dans un dispositif haptique multimodal conforme à l'invention. Ladite cellule se distingue de celle du premier mode en ce qu'elle comprend en outre un module Peltier 110' supplémentaire disposé de telle que façon que l'élément vibratoire 150 se trouve entre les deux modules Peltier 110, 110'.

Comme pour le premier mode de réalisation, la hauteur de l'élément vibratoire est inférieure aux hauteurs des deux modules Peltier et ce, pour améliorer les performances des éléments, et notamment de l'élément vibratoire. Cette variation de hauteur est répercutée par une variation des hauteurs des deux couches d'encapsulation, et notamment de la couche inférieure 501. Une topologie particulière en résulte. Cette topologie peut être avantageusement utilisée pour favoriser l'application des éléments Peltier au contact de la peau. En outre, cette topologie particulière permet de laisser une liberté de déplacement à l'élément piézoélectrique pour assurer ses vibrations. Le contact peut être amélioré en utilisant par exemple à un élément de pression 800, telle qu'une couche de polymère ou de plastique, éventuellement élastique, utilisée pour maintenir l'ensemble de la cellule en prenant appui sur les éléments Peltier, en exerçant une pression sur la surface supérieure des éléments Peltier et en exerçant ainsi une pression contre la peau (pression qui peut varier). Ceci permet de favoriser un meilleur contact thermique de la cellule avec la peau et de favoriser le comportement vibratoire de la membrane formée par le matériau encapsulant actionnée par l'élément piézoélectrique.

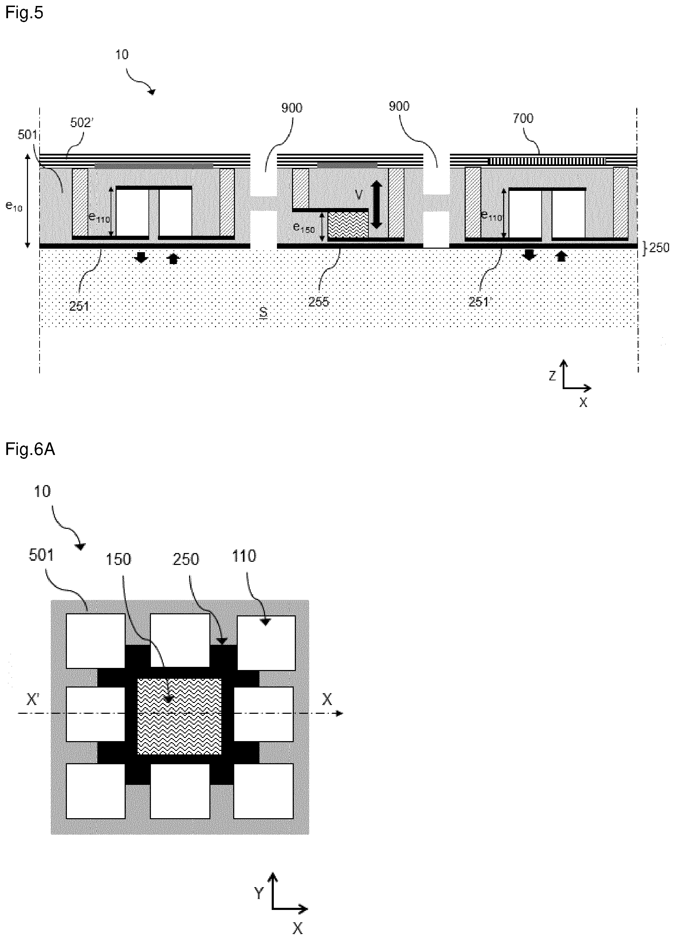

La figure 5 représente un cinquième mode de réalisation d'un dispositif haptique multimodal, représenté posé sur la peau S, plus précisément d'une cellule comprise dans un dispositif haptique multimodal, qui se distingue des autres modes de réalisation en ce que la couche d'encapsulation 501 est amincie (amincissements 900) entre les éléments (ici entre chaque module Peltier et l'élément vibratoire) de la cellule. Cela permet de favoriser la flexibilité de la cellule. Ceci peut être appliqué pour tout mode de réalisation, ou plus largement entre au moins deux éléments d'une cellule comprise dans un dispositif haptique multimodal conforme à l'invention.

On voit dans la figure 5 que, comme dans la figure 3, les pistes conductrices supérieures sont représentées intégrées dans une couche de redistribution des interconnexions, formant la couche d'encapsulation supérieure 502' flexible, caractéristique qui peut être appliquée pour tout autre mode de réalisation, ou plus largement pour un dispositif haptique multimodal conforme à l'invention.

En outre, on peut voir que dans ce mode de réalisation, une série 250 de pistes chauffantes complémentaires 251, 255, 251' est insérée sous les éléments, et sous les pistes conductrices inférieures 200. Elles permettent de réaliser des résistances chauffantes sous chacun des éléments de la cellule ainsi mises en oeuvre sur presque toute la surface au contact de la peau pour une meilleure résolution. Comme pour la piste chauffante de la figure 2, les pistes chauffantes 250 peuvent être en graphite (C) ou en métal, par exemple en argent (Ag).

Cette caractéristique est indépendante de l'amincissement du matériau encapsulant, et peut être appliquée pour tout autre mode de réalisation, ou plus largement pour un dispositif haptique multimodal conforme à l'invention, sans que ce soit nécessairement en combinaison avec la caractéristique d'amincissement du matériau encapsulant. En outre, on peut insérer des pistes chauffantes complémentaires non nécessairement sous tous les éléments, mais cela peut être inséré sous un ou quelques-uns parmi les éléments constituant une cellule.

Les différents modes de réalisation présentés jusqu'à présent représentent des éléments disposés les uns à côté des autres dans la direction longitudinale X d'une cellule. Mais cette disposition n'est pas limitative et les éléments vibratoires et thermiques peuvent être positionnés de différentes manières dans le plan XY de la cellule. En d'autres termes, dans tous les modes de réalisation présentés jusqu'à présent, les éléments peuvent être disposés autrement que selon un alignement dans la direction longitudinale X.

De préférence, le positionnement des différents éléments est optimisé pour amplifier la sollicitation vibratoire grâce à une disposition des éléments thermiques, en particulier d'éléments et/ou de modules Peltier, autour d'un élément vibratoire ou plusieurs éléments vibratoires.

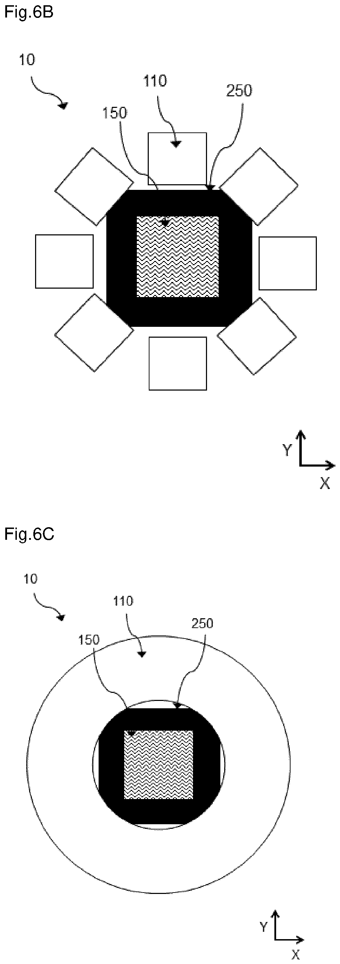

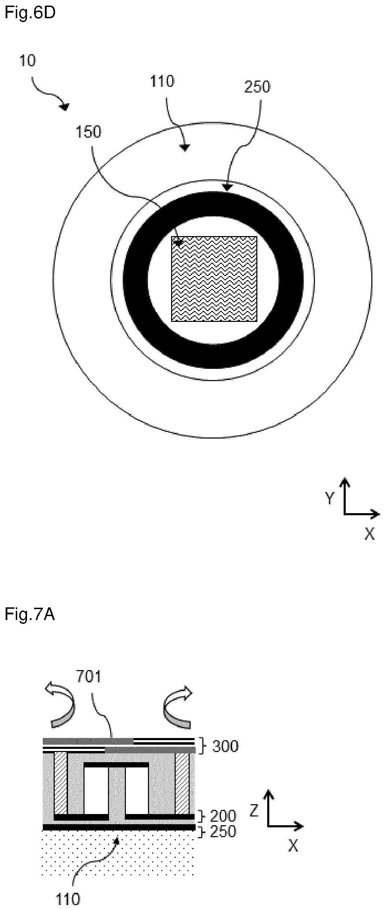

Les figures 6A à 6D présentent plusieurs exemples de configurations des éléments vibratoire (un élément piézoélectrique dans les exemples) et thermiques (plusieurs modules Peltier dans les exemples) dans le plan XY de la cellule. Les connexions électriques ne sont pas représentées sur ces figures, mais des pistes chauffantes complémentaires 250 permettant de réaliser des résistances chauffantes sont représentées. Dans les quatre configurations représentées, il y a un seul élément piézoélectrique 150, et plusieurs modules Peltier 110 sont disposés autour de cet élément piézoélectrique, soit en forme de carré (figure 6A) soit en forme de cercle, le module Peltier formant un anneau autour de l'élément piézoélectrique (figures 6B à 6D).

Le positionnement des modules Peltier autour de l'élément piézoélectrique présente plusieurs avantages :
- la masse des modules Peltier permet de former un ancrage autour de l'élément piézoélectrique pour assurer le fonctionnement vibratoire de ce dernier ;
- la surface ainsi occupée par les modules Peltier permet d'augmenter leur effet pour renforcer la sensation thermique ; elle s'accorde aussi avec une résolution des sensations thermiques de la peau généralement inférieure à celle des sensations mécaniques ;
- le couplage entre les deux fonctions (vibratoire et thermique) ainsi réalisée permet de moduler la perception, et d'accentuer les effets cognitifs.

De manière plus générale, le couplage des deux fonctions (vibratoire et thermique) peut permettre par exemple d'amplifier la perception et l'interprétation par le cerveau d'un stimulus, notamment d'une stimulation thermique en présence d'une sensation de contact.

Dans l'ensemble des modes présentés, et plus largement dans le cadre de l'invention, les différentes pistes conductrices inférieures ne présentent pas nécessairement les mêmes épaisseurs. Il en est de même pour les différentes pistes conductrices supérieures. En outre, les différentes pistes conductrices inférieures peuvent être à des hauteurs différentes en fonction de l'élément auquel elles sont associées, de même pour les différentes pistes conductrices supérieures. Les pistes conductrices connectant les éléments Peltier peuvent être plus épaisses que les pistes conductrices connectant l'élément vibratoire, et ce, pour les pistes inférieures et/ou pour les pistes supérieures. Les pistes conductrices inférieures et supérieures ne sont pas nécessairement en les mêmes matériaux. Plus généralement dans le cadre de l'invention, les caractéristiques des connexions électriques peuvent être spécifiques aux différents éléments.

Nous allons détailler maintenant des éléments et modules Peltier et des éléments piézoélectriques pouvant être mis en oeuvre pour chacun des modes de réalisation présentés, et plus généralement pour un dispositif haptique multimodal selon l'invention.

### Eléments et modules Peltier

Pour obtenir une résolution spatiale suffisante tant pour la sollicitation thermique que vibratoire, les dimensions des éléments Peltier sont de préférence limitées à quelques millimètres maximum, voire inférieures au millimètre.

On utilise de préférence plusieurs éléments thermoélectriques ou éléments Peltier (« Thermoelectric Leg » en anglais), et notamment une pluralité d'éléments N et P reliés entre eux et dont les hauteurs sont comprises entre 0,2 et 5 mm, de préférence 0,2 à 1 mm, et les sections sont comprises entre 0,2 et 4 mm² (typiquement 1 mm²). Les éléments Peltier peuvent être réalisés à base de Tellurure de Bismuth qui permet de fonctionner à des températures entre -10 à 100°C, ou à base de siliciures (MgSiSn, MnSi, SiGe, ....).

Pour une facilité d'intégration dans une cellule, le ou les deux éléments Peltier sont de préférence préalablement intégrés en module, c'est-à-dire préassemblés sur un substrat isolant recouvert de pistes conductrices (cuivre ou argent typiquement). On utilise classiquement un substrat mince en nitrure d'aluminium (AIN), en alumine (Al₂O₃), en Zircone (ZrO₂), voire en Kapton^{®} (film de polyimide) ce dernier permettant de garantir une flexibilité du module Peltier. Lorsque le substrat n'est pas flexible, le module Peltier peut être rendu flexible, par exemple par un assemblage d'éléments entre eux avec un matériau flexible. De préférence, la hauteur totale d'un module (substrat, éléments et pistes) est inférieure à 2 millimètres.

Dans les différents modes de réalisation présentés, et plus généralement pour un dispositif haptique multimodal selon l'invention, les éléments présents sont pilotés électriquement via les pistes conductrices supérieures qui permettent de leur appliquer des tensions et/ou des courants de pilotage. Ces pilotages sont réalisés de façon à générer localement des sensations de toucher vibratoires et thermiques. De préférence, le pilotage est dynamique, c'est-à-dire seuls les éléments situés sur les zones à stimuler sont activés électriquement ; ainsi cette zone peut être déplacée et sa surface modulée dans le temps, donnant ainsi une impression réaliste de stimulation en mouvement sur la surface de la peau.

Chaque module Peltier peut être piloté par l'application d'un courant allant de quelques dizaines de µAmpères jusqu'à quelques Ampères en fonction des matériaux et de la géométrie des modules (notamment taille et nombre d'éléments).

Un module Peltier peut être couplé à un thermocouple, une thermistance ou tout autre moyen apte à mesurer la température au contact de la peau lorsque le dispositif haptique est appliqué sur la peau d'une personne, la température mesurée pouvant rentrer dans une boucle d'asservissement afin de déterminer le courant à appliquer en entrée du module.

Selon la configuration du module Peltier et éventuellement du dissipateur thermique associé au module, la différence de température appliquée peut aller de quelques degrés pendant un temps relativement court jusqu'à quelques dizaines de degrés en continu (typiquement 20°C). Pour une sensation thermique de fort refroidissement prolongé au-delà de plusieurs minutes, un dissipateur doit être utilisé pour évacuer la chaleur. Sans dissipateur ou avec un dissipateur très mince, la sensation thermique de froid sera transitoire et durera de quelques secondes à la minute environ.

Une cellule peut comprendre en outre des moyens thermiques complémentaires associés aux éléments thermiques, comme un dissipateur thermique (repéré par la référence 700 dans la figure 3) et/ou des moyens mécaniques complémentaires associés à l'élément vibratoire, tel un raidisseur, le tout, afin d'améliorer les performances. Ces moyens thermiques et/ou mécaniques complémentaires peuvent consister en des éléments supplémentaires ou peuvent être obtenus par une intégration et un placement optimisé des éléments déjà existants.

Différents dissipateurs thermiques sont représentés dans les figures 7A à 7C. ces dissipateurs thermiques sont avantageusement mis en oeuvre pour garantir un fonctionnement optimal en mode refroidissement d'un module Peltier.

Dans la figure 7A, le dissipateur thermique est un diffuseur thermique 701 intégré ou déjà existant dans les pistes conductrices supérieures 300, et plus précisément dans la piste conductrice située au-dessus du module Peltier. Le diffuseur peut être en métal ou en carbone.

Un tel diffuseur ainsi configuré permet d'améliorer la convection naturelle en permettant d'une part une meilleure répartition de la chaleur et d'autre part une augmentation de la surface d'échange de la chaleur avec l'extérieur. En régime permanent, elle peut apporter une différence de température de quelques °C, et en régime transitoire, elle peut apporter une différence de température plus importante sur une échelle de temps inférieure à 1 seconde.

Cette solution est une des solutions préférées lorsque le module Peltier et/ou la cellule est flexible.

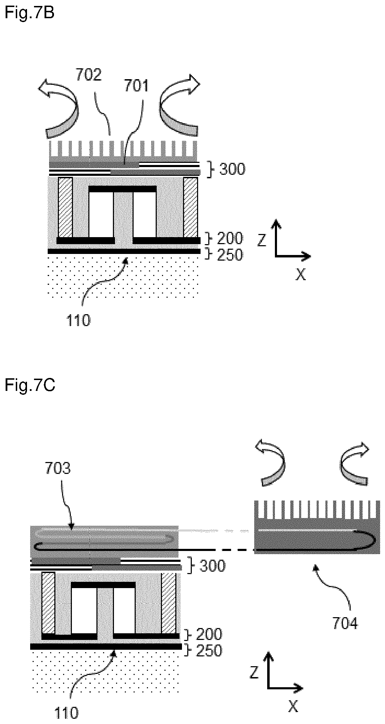

Dans la figure 7B, un élément de dissipation externe passif 702, par exemple un bloc de cuivre ou d'aluminium à ailettes, est ajouté sur les pistes conductrices supérieures 300, au moins sur la piste conductrice située au-dessus du module Peltier pour permettre d'améliorer la convection naturelle. Tel que représenté, l'élément de dissipation à ailettes est en contact avec le diffuseur thermique 701.

Un tel élément de dissipation externe permet de favoriser le stockage de la chaleur absorbée par le module Peltier (stockage sous forme de chaleur massique) et son évacuation dans l'air ambiant par convection naturelle. En régime permanent, elle peut apporter une différence de température de plusieurs °C, et en régime transitoire, elle peut apporter une différence de température plus importante sur échelle de temps de quelques secondes.

Cette solution est toutefois limitée lorsque le module et/ou la cellule est flexible, sauf si elle est déportée en dehors de la zone flexible.

Dans la figure 7C, un circuit microfluidique 703, éventuellement flexible, est disposé au-dessus du module Peltier, sur les pistes conductrices supérieures 300, au moins sur la piste conductrice située au-dessus du module Peltier. Ceci permet d'améliorer encore plus la dissipation thermique, par conversion forcée, en extrayant la chaleur à l'aide d'un fluide caloporteur lors du refroidissement. La chaleur extraite est ensuite acheminée vers un échangeur de chaleur 704 pour refroidir le liquide caloporteur par convection naturelle. Un tel dispositif peut être conçu pour être portable. En régime permanent, elle peut apporter une différence de température de plusieurs dizaines de °C.

### Eléments vibratoires

Les éléments vibratoires peuvent être des éléments piézoélectriques, des éléments ferroélectriques et/ou électromagnétiques. Les géométries de ces éléments vibratoires peuvent être variées. On peut par exemple utiliser des éléments de forme parallélépipédiques à section carrée, de côté compris entre 0,5 et 10 mm, de préférence 1 mm. L'épaisseur peut être comprise entre 1 µm et 1 mm, de préférence entre 50 et 100 µm. Les tensions à appliquer au matériau seront d'autant plus fortes que l'épaisseur est forte, pour obtenir un champ électrique et donc un effet vibratoire équivalent.

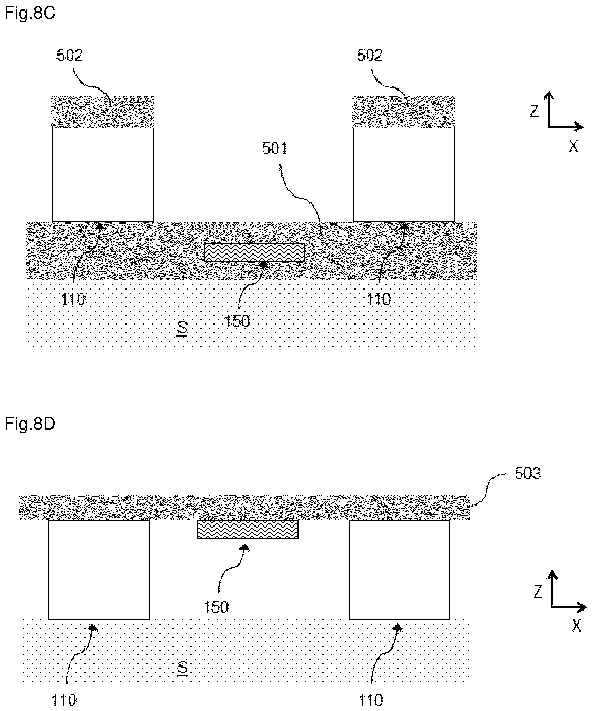

Les figures 8A, 8B, 8C et 8D représentent quatre variantes correspondant à la configuration d'une cellule selon la figure 6A c'est-à-dire avec un élément piézoélectrique 150 au centre et huit modules Peltier 110 autour mais sans la résistance 250, vue en coupe selon l'axe XX' et appliquée sur une peau S. Les différentes variantes permettent d'exploiter la présence des modules Peltier autour de l'élément piézoélectrique pour améliorer le fonctionnement et la réponse vibratoire de l'élément piézoélectrique.

La figure 8A représente une première variante dans laquelle la couche d'encapsulation 501 permet d'ancrer les parties inférieures des modules Peltier et de l'élément piézoélectrique, mais elle ne les encapsule pas entièrement.

L'épaisseur de la couche d'encapsulation est de 150 µm et ses dimensions dans le plan XY sont de 5*5 mm².

La figure 8B représente une deuxième variante qui diffère de la première variante en ce qu'elle comprend en outre un élément de pression 800, telle qu'une couche de polymère ou de plastique, éventuellement élastique, utilisée pour maintenir l'ensemble de la cellule en prenant appui sur les éléments Peltier, en exerçant une pression sur la surface supérieure des éléments Peltier et en exerçant ainsi une pression contre la peau (pression qui peut varier). Ceci permet de favoriser un meilleur contact thermique de la cellule avec la peau et de favoriser le comportement vibratoire de la membrane formée par le matériau encapsulant actionnée par l'élément piézoélectrique.

L'épaisseur de la couche d'encapsulation est de 150 µm et ses dimensions dans le plan XY sont de 5*5 mm².

La figure 8C représente une troisième variante qui diffère de la première variante en ce que l'élément piézoélectrique est entièrement encapsulé dans la première couche d'encapsulation 501 qui présente une épaisseur de 650 µm (150 µm + 500 µm). Les modules Peltier sont ancrés en partie inférieure par la première couche d'encapsulation 501 et en partie supérieure par une deuxième couche d'encapsulation 502 de 500 µm d'épaisseur.

Les dimensions des couches d'encapsulation dans le plan XY sont de 5*5 mm².

La figure 8D représente une quatrième variante qui diffère de la première variante en ce que l'élément piézoélectrique et les modules Peltier sont ancrés en partie supérieure par une couche d'encapsulation 503 de 150 µm d'épaisseur.

Les dimensions de la couche d'encapsulation 503 dans le plan XY sont de 5*5 mm².

Des simulations par un logiciel de simulation numérique COMSOL ont été réalisées pour les différentes variantes.

Les simulations ont été réalisées avec des modules Peltier formant chacun un cube de 1 mm³ et l'élément piézoélectrique un parallélépipède de 1 mm² sur une hauteur de 100 µm. Dans les simulations, les modules Peltier sont régulièrement espacés entre eux et avec l'élément piézoélectrique, avec une distance de 500 µm entre deux modules Peltier adjacents, et entre l'élément piézoélectrique et les modules Peltier les plus proches. Les dimensions de la première ou de la deuxième couche d'encapsulation dans le plan XY sont de 5*5 mm² et l'épaisseur est de 150 µm ou 650 µm comme indiqué plus avant.

Le matériau encapsulant est de préférence flexible. Il peut être un polymère flexible. Les simulations ont été réalisées avec un polyimide.

Ceci n'est pas limitatif : les modules Peltier et l'élément piézoélectrique peuvent avoir les mêmes dimensions dans le plan XY (et des hauteurs différentes) ou des dimensions différentes dans le plan XY. De même, les différents modules Peltier peuvent également avoir les mêmes dimensions ou des dimensions différentes (dans le plan XY et/ou en hauteur Z). En outre, l'espacement dans une direction donnée X et/ou Y entre deux modules Peltier adjacents est fonction de la dimension desdits éléments dans ladite direction. L'espacement dans une direction donnée X et/ou Y peut être compris dans une gamme allant de 1/10 à 2 fois la dimension de l'élément dans ladite direction. En outre, l'élément piézoélectrique peut s'étendre sur toute la surface laissée libre par les modules Peltier au centre de la cellule ou s'étendre sur une partie de cette surface. Préférentiellement, la moitié de la dimension de l'élément piézoélectrique dans la direction X, respectivement dans la direction Y, est comprise entre 50 et 60 % de la distance entre l'élément piézoélectrique et les modules Peltier les plus proches dans la direction X, respectivement la direction Y. Le matériau encapsulant peut être en un autre polymère flexible voire n'est pas nécessairement flexible.

Les simulations montrent que la couche d'encapsulation forme une membrane qui peut transmettre la vibration générée par l'élément piézoélectrique, ce qui génère un effet vibrotactile sur la peau (qui peut être accompagné d'un effet thermique par les modules Peltier). En outre, les modules Peltier avec le matériau d'encapsulation forment un raidisseur pour l'actionnement de l'élément piézoélectrique, améliorant ainsi le fonctionnement vibratoire.

Les inventeurs ont ainsi démontré un effet de synergie entre les modules Peltier et l'élément vibratoire, les modules Peltier ne servant pas seulement à produire du chaud ou du froid, mais contribuant en outre au fonctionnement de l'élément vibratoire. Il est à noter que ceci peut être vrai pour tout autre élément thermique, autre qu'un élément Peltier, dans la mesure où il présente une hauteur supérieure à l'élément vibratoire. Ceci permet en outre d'éviter de rajouter un élément mécanique complémentaire associé à l'élément vibratoire, tel un raidisseur.

L'amplitude de vibration dépend de plusieurs paramètres dont la taille de la membrane formée par la couche d'encapsulation, et le type d'élément vibratoire. Les inventeurs ont déterminé qu'une amplitude de l'ordre de 10 µm (compatible avec un effet haptique vibrotactile) peut aisément être atteindre sous une tension de l'ordre 10V avec une membrane/couche d'encapsulation de 1cm².

La fréquence de résonance de la cellule dépend de plusieurs paramètres dont l'épaisseur de la membrane/couche d'encapsulation et en particulier l'épaisseur d'encapsulation autour de l'élément piézoélectrique comme illustré dans la figure 8C. Les inventeurs ont déterminé que la fréquence de résonance de la cellule était de 244kHz pour une épaisseur de 650µm (figure 8C), contre 77kHz pour une épaisseur de 150µm (figure 8A ou figure 8B).

La fréquence de résonance de la cellule dépend également des dimensions dans le plan de la membrane/couche d'encapsulation. Les inventeurs ont déterminé qu'en augmentant les dimensions de la couche d'encapsulation, on diminue la fréquence de résonance. La simulation a permis de montrer qu'avec des dimensions de 7*7 mm² (et avec un élément piézoélectrique de 3,5*3,5 mm² et 20 modules Peltier) la fréquence de résonance était de 31.5kHz et qu'avec des dimensions de 20*20 mm² (et avec un élément piézoélectrique de 10*10 mm² et 56 modules Peltier) la fréquence de résonance était de 3.8kHz.

La simulation de la quatrième variante de la figure 8D donne une fréquence de 77kHz. Cette quatrième variante permet de transmettre vers la surface de la peau une onde acoustique.

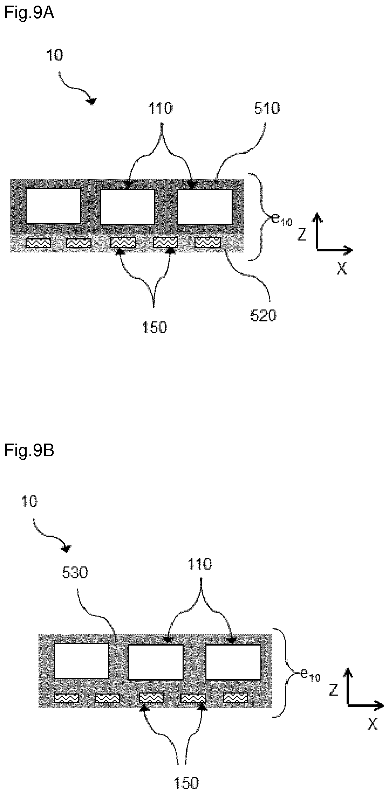

Les figures 9A et 9B représentent un sixième mode de réalisation d'un dispositif haptique multimodal, dans lequel les éléments ne sont pas disposés les uns à côtés des autres dans le plan XY, mais l'au-dessus de l'autre dans la direction Z perpendiculaire au plan. Les figures 9A et 9B représentent précisément des éléments thermiques (module Peltier notamment) disposés au-dessus d'éléments vibratoires, et assemblés.

Dans une première variante (figure 9A), les éléments thermiques 110 sont encapsulés dans une première couche d'encapsulation 510, et les éléments vibratoires 150 sont encapsulés dans une deuxième couche d'encapsulation 520, les deux couches étant en un même matériau ou en deux matériaux différents. Les deux couches sont assemblées, par exemple par lamination desdites couches. Les couches peuvent être flexibles, par exemple en un polymère flexible. Chacune des couches peut inclure les connexions électriques permettant d'alimenter et de piloter les différents éléments.

Dans une deuxième variante (figure 9B), les éléments thermiques 110 et les éléments vibratoires 150 sont encapsulés dans une même couche d'encapsulation 530.

Ce sixième mode de réalisation peut être développé sous une pluralité de variantes qui ne peuvent pas toutes être développées, avec des éléments thermiques qui ne sont pas seulement des éléments Peltier, avec un ou plusieurs éléments vibratoires, avec une ou plusieurs couches supplémentaires... En outre, ce mode de réalisation peut être combiné avec les autres modes de réalisation, en ce que les éléments peuvent être disposés les uns à côtés des autres dans le plan XY et aussi dans la direction Z. Toutes les combinaisons techniquement possibles peuvent être envisagées.

Plusieurs cellules, notamment les cellules décrites selon les différents modes de réalisation, peuvent être assemblées les unes à côté des autres dans le plan XY sous forme de matrice, ce qui permet d'obtenir un dispositif haptique de plus grande surface. Il est possible d'assembler les mêmes types de cellules ou des cellules différentes.

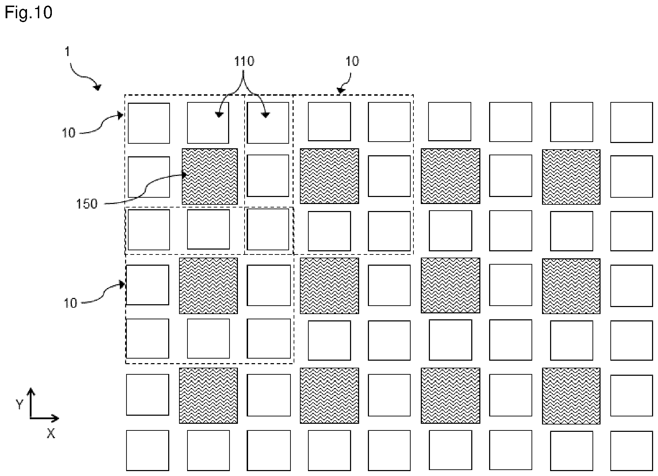

La figure 10 représente une matrice constituée de plusieurs cellules, la matrice étant comprise dans un dispositif haptique multimodal. La matrice 1 est représentée de manière très schématique sans les connexions électriques nécessaires pour alimenter et piloter les différents éléments. La matrice représentée comprend une pluralité de cellules de base identiques 10. La cellule de base correspond à la cellule de la figure 6A (un élément piézoélectrique 150 central et huit modules Peltier 110 régulièrement disposés autour) qui est répétée régulièrement plusieurs fois. En outre, deux cellules adjacentes ont en commun une rangée de trois modules Peltier.

La matrice représentée comprend plusieurs cellules identiques disposées régulièrement les unes à côté des autres dans le plan XY et elles sont encapsulées dans une même couche d'encapsulation. Mais cette configuration n'est pas limitative. Ainsi une matrice peut être composée de :
- plusieurs cellules identiques ou différentes ; et/ou
- plusieurs cellules disposées de manière régulière ou irrégulière dans le plan XY ; et/ou
- des cellules avec des couches d'encapsulation différentes ; et/ou
- un ou plusieurs éléments disposés l'un au-dessus de l'autre dans la direction Z perpendiculaire au plan XY ;
- une ou plusieurs cellules disposées l'une au-dessus de l'autre dans la direction Z perpendiculaire au plan XY.

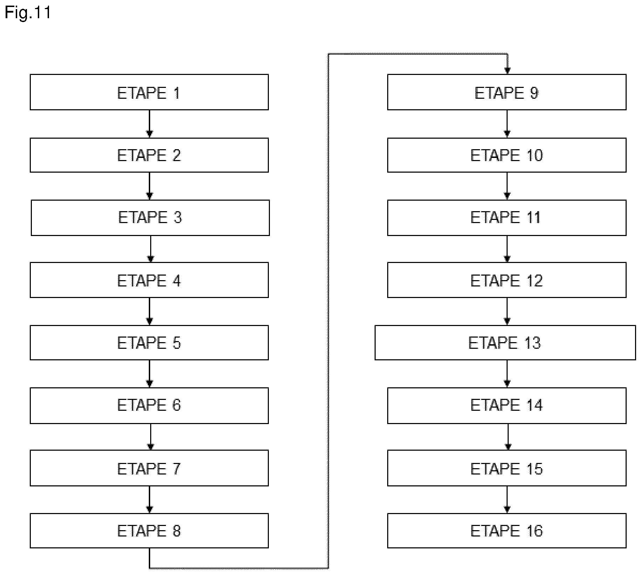

La Fig.11 représente un exemple de procédé de réalisation d'un dispositif haptique multimodal, et plus particulièrement d'une cellule comprise dans un dispositif. Concernant les références des éléments dans une cellule, on peut se référer aux figures précédentes et notamment aux figures 1 à 5 :
- Etape 1 : on part d'un substrat en un matériau isolant qui peut être un substrat flexible ou étirable en polymère (PET, PEN, PI, PC, silicone...), un verre fin ou encore un métal fin ; le substrat peut être obtenu sous forme de film sec ou de film liquide solidifié ;
- Etape 2 (optionnelle) : on dépose une ou des pistes chauffantes 250 ; il peut s'agir d'un métal ou d'une encre conductrice (par exemple à base d'argent ou de carbone) déposé(e) puis structuré(e) par lithographie ou impression ;
- Etape 3 (optionnelle) : on dépose un film en un matériau isolant ; il peut s'agir d'un des matériaux cités pour le substrat, voire le même que le matériau du substrat, et il peut être obtenu sous forme de film sec ou de film liquide solidifié ;

- Etape 4 : on réalise les pistes conductrices inférieures 200 ; il peut s'agir d'un métal ou d'une encre conductrice (par exemple à base d'argent ou de carbone) déposé(e) puis structuré(e) par lithographie ou impression ;
- Etape 5 : on reporte le ou les éléments vibratoires 150 en contact avec les pistes conductrices inférieures correspondantes 205 ;
- Etapes 6 et 7 : on reporte le ou les éléments thermiques 111, 112, 110, 130, 140 en contact avec les pistes conductrices inférieures correspondantes 201, 202, 203, 204 : par exemple au moins un élément Peltier 111 de type P et au moins un élément Peltier 112 de type N pour former un module Peltier 110, une résistance chauffante 130 et/ou un élément radiant 140 ;
- Etape 8 : on dépose une couche d'un matériau isolant ; il peut s'agir d'un des matériaux cités pour le substrat, voire le même que le matériau du substrat ; ce dépôt peut être réalisé sous forme liquide par exemple par une des méthodes « spin coating » ou « slot die » connues de l'homme du métier ; cela permet de finaliser la première couche en matériau isolant (première couche d'encapsulation 501) ;
- Etape 9 (facultative) : on peut prévoir une étape de planarisation de la couche en matériau isolant déposée lors de l'étape 8, par exemple par un polissage mécanochimique (en anglais, Chemical Mechanical Polishing ou CMP) ;
- Etape 10 : on ouvre les vias 400 dans la couche en matériau isolant déposée lors de l'étape 8, en utilisant par exemple une méthode de lithographie et gravure chimique ou plasma, ou une méthode laser ;
- Etape 11 : on remplit les vias en un matériau conducteur (par exemple cuivre) pour réaliser les connexions électriques : il peut s'agit d'une méthode de croissance (méthode « electroless » ou méthode de dépôt de pâte conductrice par sérigraphie) ;
- Etape 12 : on réalise les pistes conductrices supérieures 300 (connexion éléments Peltier et routages des différents éléments) : il peut s'agir d'un métal ou d'une encre conductrice (par exemple à base d'argent ou de carbone) déposé(e) puis structuré(e) par lithographie ou impression ;
- Etape 13 (optionnelle) : on peut réaliser des interconnexions de routage, notamment à plusieurs niveaux si nécessaire : il peut s'agir d'un métal ou d'une encre conductrice (par exemple à base d'argent ou de carbone) déposé(e) puis structuré(e) par lithographie ou impression ;
- Etape 14 : on réalise la deuxième couche en matériau isolant (deuxième couche d'encapsulation 502) ; il peut s'agir d'un des matériaux cités pour le substrat, voire le même que le matériau du substrat, et il peut être appliqué sous forme de film sec ou de film liquide solidifié ;
- Etape 15 : on réalise les ouvertures des pads 600 dans la deuxième couche, pour permettre les connexions de pilotage de l'ensemble des éléments, en utilisant par exemple une méthode de lithographie et gravure chimique ou plasma, ou une méthode laser ;
- Etape 16 (optionnelle) : on peut réaliser des amincissements 900 d'une ou des couches en matériau isolant, par exemple par une méthode de lithographie et gravure chimique ou plasma, ou par une méthode laser.

Concernant les étapes 1, 3 et 8, elles forment la première couche d'encapsulation telle que décrite précédemment, mais en plusieurs étapes afin de pouvoir supporter, introduire et recouvrir les éléments et les pistes conductrices. On voit que cette première couche peut être en un seul matériau isolant ou en plusieurs différents matériaux isolants.

Concernant l'étape 5, il peut s'agit d'élément(s) piézoélectrique(s) ou ferroélectriques, voire électromagnétiques, par exemple d'éléments à base de PZT (Titano-Zirconate de Plomb) ou tout autre matériau adapté ; le report peut être réalisé par une méthode de type « pick and place » (prise d'un élément sur un support source par un bras motorisé, positionnement et placement de l'élément sur le substrat).

Concernant les étapes 6 et 7, les éléments Peltier peuvent être réalisés à base de Tellurure de Bismuth qui permet de fonctionner à des températures entre - 10 à 100°C, ou à base de siliciures (MgSiSn, MnSi, SiGe, ....) matériaux avec des performances inférieures sur cette gamme de température mais dont les ressources sont moins limitées.

Les éléments Peltier peuvent être préalablement intégrés en modules, c'est-à-dire préassemblés sur un substrat isolant recouvert de pistes conductrices (cuivre ou argent typiquement). Ainsi, les éléments Peltier peuvent être réalisés à base de matériaux massifs reportés par une méthode « pick and place » sur des substrats isolants de type DBC (« Direct Bonded Copper » en anglais), tels que le nitrure d'aluminium (AIN), l'alumine (Al₂O₃), la Zircone (ZrO₂), voire le Kapton^{®} (film de polyimide), ou par des techniques de dépôts fins ou épais (quelques dizaines à quelques centaines de micromètres).

De préférence, on utilise des éléments massifs amincis (typiquement entre 200 et 600 µm) pour améliorer leurs performances. Ces éléments peuvent également être déposés ou imprimés en films (composites nanotubes de carbone /polymères, super-réseaux TiS₂/hexylamine.

Ainsi, une cellule peut être aisément fabriquée par des méthodes de fabrication connues de microélectronique. Plusieurs cellules peuvent être réalisées en même temps au cours des étapes précédemment décrites, afin de former une matrice de plusieurs cellules.

Les différents modes présentés peuvent être combinés entre eux.

En outre, la présente invention n'est pas limitée aux modes de réalisation précédemment décrits mais s'étend à tout mode de réalisation entrant dans la portée des revendications.

## Revendications

1. Dispositif haptique multimodal comprenant une matrice (1) intégrant au moins une cellule (10), chaque cellule comportant :
- au moins un élément thermique (110, 111, 112, 130, 140, 110') apte à générer un refroidissement et un chauffage ;
- au moins un élément vibratoire (130) apte à générer une vibration ; et
- au moins une couche d'encapsulation (500, 501, 502, 502', 503, 510, 520, 530) en un matériau électriquement isolant ;
le au moins un élément vibratoire et le au moins un élément thermique étant ancrés en au moins une partie dans ladite au moins une couche d'encapsulation ;
chaque cellule (10) étant adaptée pour être en contact directement ou indirectement avec la peau d'une personne de manière à lui transmettre des sensations thermique et/ou vibratoire, ladite cellule présentant une épaisseur inférieure ou égale à dix millimètres, le au moins un élément thermique présentant une hauteur supérieure à celle du au moins un élément vibratoire,
au moins une cellule (10) comprenant :
- un élément vibratoire (150) central et ;
- au moins deux modules Peltier (110, 110') disposés autour dudit élément vibratoire et formant ledit au moins un élément thermique.

2. Dispositif selon la revendication 1, au moins une cellule (10) comprenant en outre :
- une pluralité (200) de pistes conductrices inférieures (201, 202, 203, 204, 205), aptes à réaliser les connexions électriques au niveau des parties inférieures des éléments thermique et vibratoire ; et
- une pluralité (300) de pistes conductrices supérieures (301, 303, 304, 305), aptes à réaliser les connexions électriques au niveau des parties supérieures des éléments thermique et vibratoire, et de préférence au moins le routage pour le pilotage desdits éléments.

3. Dispositif selon la revendication 2, la au moins une cellule (10) comprenant en outre une pluralité de vias (500) conducteurs, lesdits vias permettant de réaliser des connexions électriques verticales entre les pistes conductrices supérieures et les éléments thermique et vibratoire.

4. Dispositif selon l'une quelconque des revendications précédentes, la au moins une couche d'encapsulation (500) comprenant une première couche d'encapsulation (501, 510) en un premier matériau isolant et une deuxième couche d'encapsulation (502, 502', 520) en un deuxième matériau isolant, les première et deuxième couches pouvant être jointes, et/ou les premier et deuxième matériaux isolants pouvant être identiques.

5. Dispositif selon l'une des revendications 2 ou 3 en combinaison avec la revendication 4, la deuxième couche d'encapsulation (502') intégrant les pistes conductrices supérieures, formant une couche de redistribution des interconnexions, ladite couche de redistribution étant de préférence flexible.

6. Dispositif selon l'une quelconque des revendications 1 à 5, au moins un élément thermique étant un élément Peltier, de préférence au moins deux éléments Peltier de type N et P (111, 112) connectés électriquement en série pour former un module Peltier (110).

7. Dispositif selon la revendication 6, au moins une cellule (10) comprenant un moyen de dissipation thermique (700, 701, 702, 703) associé à au moins un module Peltier (110).

8. Dispositif selon l'une quelconque des revendications précédentes, au moins un élément thermique étant une résistance chauffante (130) et/ou un élément radiant (140), par exemple un élément radiant Infrarouge (IR).

9. Dispositif selon l'une quelconque des revendications précédentes, au moins un élément vibratoire (150) étant un élément piézoélectrique, un élément ferroélectrique et/ou un élément électromagnétique.

10. Dispositif selon l'une quelconque des revendications précédentes, au moins un élément thermique et/ou vibratoire, de préférence tous les éléments thermique et/ou vibratoire, étant entièrement encapsulé(s) dans au moins une couche d'encapsulation.

11. Dispositif selon l'une quelconque des revendications précédentes, la au moins une couche d'encapsulation étant en un ou plusieurs des matériaux suivants : un polymère tels qu'un polytéréphtalate d'éthylène (PET), un polynaphtalate d'éthylène (PEN), un polyimide (PI), un polycarbonate (PC) ou un silicone ; un verre ; un métal ; ou encore un matériau non organique tel que l'oxyde de silicium.

12. Dispositif selon l'une quelconque des revendications précédentes, la au moins une couche d'encapsulation étant en un matériau flexible ou étirable, par exemple un polymère flexible et/ou un élastomère.

13. Dispositif selon l'une quelconque des revendications précédentes, au moins une cellule (10) comprenant en outre au moins une piste chauffante complémentaire (250) sous au moins un élément thermique et/ou vibratoire, et de préférence sous au moins une piste conductrice inférieure.

14. Dispositif selon l'une quelconque des revendications précédentes, au moins une cellule (10) comportant des amincissements (900) entre au moins deux éléments thermique et/ou vibratoire dans l'épaisseur d'au moins une couche d'encapsulation (501).

15. Dispositif selon l'une quelconque des revendications précédentes, au moins une cellule (10) comportant un élément de pression (800), notamment une couche de polymère ou de plastique, éventuellement élastique, disposée sur l'ensemble de ladite cellule en prenant appui sur au moins un élément, apte à exercer indirectement une pression contre la peau.

16. Dispositif selon la revendication 1, les modules Peltier (110, 110') présentant des hauteurs supérieures à la hauteur de l'élément vibratoire (150).

17. Dispositif selon l'une quelconque des revendications précédentes, la matrice (1) comprenant plusieurs cellules (10), de préférence plusieurs cellules identiques.

18. Dispositif selon l'une quelconque des revendications précédentes, au moins deux éléments thermique et/ou vibratoire étant disposés l'un au-dessus de l'autre dans une même cellule et/ou une matrice de cellules.

19. Dispositif selon l'une quelconque des revendications précédentes, au moins deux éléments thermique et/ou vibratoire étant disposés l'un à côté de l'autre dans une même cellule et/ou une matrice de cellules.

## Patentansprüche

1. Multimodale haptische Vorrichtung, umfassend eine Matrix (1), in der mindestens eine Zelle (10) integriert ist, wobei jede Zelle Folgendes aufweist:
- mindestens ein Thermoelement (110, 111, 112, 130, 140, 110'), das in der Lage ist, ein Kühlen und ein Heizen zu erzeugen;
- mindestens ein Vibrationselement (130), das in der Lage ist, eine Vibration zu erzeugen; und
- mindestens eine Einkapselungsschicht (500, 501, 502, 502', 503, 510, 520, 530) aus einem elektrisch isolierenden Material;
wobei das mindestens eine Vibrationselement und das mindestens eine Thermoelement in mindestens einem Teil in der mindestens einen Einkapselungsschicht verankert ist;
wobei jede Zelle (10) dafür geeignet ist, derart direkt oder indirekt mit der Haut einer Person in Kontakt zu sein, dass dieser thermische und/oder vibrierende Empfindungen übertragen werden, wobei die Zelle eine Dicke aufweist, die kleiner als oder gleich zehn Millimeter ist, wobei das mindestens eine Thermoelement eine Höhe aufweist, die größer als diejenige des mindestens einen vibrierenden Elements ist,
mindestens eine Zelle (10), umfassend:
- ein zentrales Vibrationselement (150) und
- mindestens zwei Peltier-Module (110, 110'), die um das Vibrationselement herum angeordnet sind und das mindestens eine Thermoelement bilden.

2. Vorrichtung nach Anspruch 1, wobei mindestens eine Zelle (10) ferner Folgendes umfasst:
- eine Vielzahl (200) von unteren leitfähigen Bahnen (201, 202, 203, 204, 205), die in der Lage sind, die elektrischen Verbindungen an den unteren Teilen der Thermoelemente und Vibrationselemente zu erzeugen; und
- eine Vielzahl (300) von oberen leitfähigen Bahnen (301, 303, 304, 305), die in der Lage sind, die elektrischen Verbindungen an den oberen Teilen der Thermoelemente und Vibrationselemente und vorzugsweise mindestens das Routing zum Antreiben der Elemente zu erzeugen.

3. Vorrichtung nach Anspruch 2, wobei die mindestens eine Zelle (10) ferner eine Vielzahl von leitfähigen Durchkontaktierungen (500) umfasst, wobei die Durchkontaktierungen das Erzeugen von vertikalen elektrischen Verbindungen zwischen den oberen leitfähigen Bahnen und dem Thermoelement und dem Vibrationselement ermöglichen.

4. Vorrichtung nach einem der vorhergehenden Ansprüche, wobei die mindestens eine Einkapselungsschicht (500) eine erste Einkapselungsschicht (501, 510) aus einem ersten isolierenden Material und eine zweite Einkapselungsschicht (502, 502', 520) aus einem zweiten isolierenden Material umfasst, wobei die erste und die zweite Schicht zusammengefügt werden können und/oder das erste und das zweite isolierende Material identisch sein können.

5. Vorrichtung nach einem der Ansprüche 2 oder 3 in Kombination mit Anspruch 4, wobei in der zweiten Einkapselungsschicht (502') die oberen leitfähigen Bahnen integriert sein können, wobei sie eine Zwischenverbindungs-Rückverteilungsschicht bilden, wobei die Rückverteilungsschicht vorzugsweise flexibel ist.

6. Vorrichtung nach einem der Ansprüche 1 bis 5, wobei es sich bei mindestens einem Thermoelement um ein Peltier-Element handelt, vorzugsweise mindestens zwei Peltier-Elemente vom Typ N und P (111, 112), die elektrisch in Reihe geschaltet sind, um ein Peltier-Modul (110) zu bilden.

7. Vorrichtung nach Anspruch 6, wobei mindestens eine Zelle (10) ein Wärmesenkenmittel (700, 701, 702, 703) umfasst, das mindestens einem Peltier-Modul (110) zugeordnet ist.

8. Vorrichtung nach einem der vorhergehenden Ansprüche, wobei mindestens ein Thermoelement ein Heizwiderstand (130) und/oder ein Strahlungselement (140), zum Beispiel ein Infrarot-Strahlungselement (IR) ist.

9. Vorrichtung nach einem der vorhergehenden Ansprüche, wobei es sich bei mindestens einem Vibrationselement (150) um ein piezoelektrisches Element, ein ferroelektrisches Element und/oder ein elektromagnetisches Element handelt.

10. Vorrichtung nach einem der vorhergehenden Ansprüche, wobei mindestens ein Thermoelement und/oder Vibrationselement, vorzugsweise alle Thermoelemente und/oder Vibrationselemente, vollständig in mindestens einer Einkapselungsschicht eingekapselt sind.

11. Vorrichtung nach einem der vorhergehenden Ansprüche, wobei die mindestens eine Einkapselungsschicht aus einem oder mehreren der folgenden Materialien besteht: einem Polymer, wie einem Polyethylenterephthalat (PET), einem Polyethylennaphthalat (PEN), einem Polyimid (PI), einem Polycarbonat (PC) oder einem Silicium; einem Glas, einem Metall oder auch einem nicht organischen Material, wie Siliciumoxid.

12. Vorrichtung nach einem der vorhergehenden Ansprüche, wobei die mindestens eine Einkapselungsschicht aus einem flexiblen oder streckbaren Material besteht, zum Beispiel einem flexiblen Polymer und/oder einem Elastomer.

13. Vorrichtung nach einem der vorhergehenden Ansprüche, wobei mindestens eine Zelle (10) ferner mindestens eine komplementäre Heizbahn (250) unter mindestens einem Thermoelement und/oder Vibrationselement und vorzugsweise unter mindestens einer unteren leitfähigen Bahn umfasst.

14. Vorrichtung nach einem der vorhergehenden Ansprüche, wobei mindestens eine Zelle (10) Einschnürungen (900) zwischen mindestens zwei Thermoelementen und/oder Vibrationselementen in der Dicke mindestens einer Einkapselungsschicht (501) aufweist.

15. Vorrichtung nach einem der vorhergehenden Ansprüche, wobei mindestens eine Zelle (10) ein Druckelement (800), insbesondere eine Polymer- oder Kunststoffschicht, wahlweise elastisch, aufweist, das auf dem Aufbau der Zelle angeordnet ist, indem es sich auf mindestens einem Element abstützt, das in der Lage ist, indirekt einen Druck auf die Haut auszuüben.

16. Vorrichtung nach Anspruch 1, wobei die Peltier-Module (110, 110') Höhen aufweisen, die höher sind als die Höhe des Vibrationselements (150).

17. Vorrichtung nach einem der vorhergehenden Ansprüche, wobei die Matrix (1) mehrere Zellen (10), vorzugsweise mehrere identische Zellen, umfasst.

18. Vorrichtung nach einem der vorhergehenden Ansprüche, wobei mindestens zwei Thermoelemente und/oder Vibrationselemente übereinander in einer selben Zelle und/oder einer Matrix von Zellen angeordnet sind.

19. Vorrichtung nach einem der vorhergehenden Ansprüche, wobei mindestens zwei Thermoelemente und/oder Vibrationselemente nebeneinander in einer selben Zelle und/oder einer Matrix von Zellen angeordnet sind.

## Claims

1. A multimodal haptic device comprising a matrix (1) incorporating at least one cell (10), each cell having:
- at least one thermal element (110, 111, 112, 130, 140, 110') capable of generating cooling and heating;
- at least one vibratory element (130) capable of generating a vibration; and
- at least one encapsulation layer (500, 501, 502, 502', 503, 510, 520, 530) made of an electrically insulating material;
the at least one vibratory element and the at least one thermal element being anchored in at least one part in said at least one encapsulation layer;
each cell (10) being adapted to be directly or indirectly in contact with the skin of a person so as to transmit thermal and/or vibratory sensations to that person, said cell having a thickness less than or equal to ten millimetres, the at least one thermal element having a height greater than that of the at least one vibratory element,
at least one cell (10) comprising:
- a central vibratory element (150); and
- at least two Peltier modules (110, 110') disposed around said vibratory element and forming said at least one thermal element.

2. The device according to claim 1, at least one cell (10) further comprising:
- a plurality (200) of bottom conductive tracks (201, 202, 203, 204, 205), capable of producing the electrical connections at the bottom parts of the thermal and vibratory elements; and
- a plurality (300) of top conductive tracks (301, 303, 304, 305), capable of producing the electrical connections at the top parts of the thermal and vibratory elements, and preferably at least the routing for the driving of said elements.

3. The device according to claim 2, the at least one cell (10) further comprising a plurality of conductive vias (500), said vias allowing vertical electrical connections to be produced between the top conductive tracks and the thermal and vibratory elements.

4. The device according to any one of the preceding claims, the at least one encapsulation layer (500) comprising a first encapsulation layer (501, 510) made of a first insulating material and a second encapsulation layer (502, 502', 520) made of a second insulating material, the first and second layers being able to be joined, and/or the first and second insulating materials being able to be the same.

5. The device according to one of claims 2 or 3 in combination with claim 4, the second encapsulation layer (502') incorporating the top conductive tracks, forming an interconnect redistribution layer, said redistribution layer being preferably flexible.

6. The device according to any one of claims 1 to 5, at least one thermal element being a Peltier element, preferably at least two Peltier elements of N and P type (111, 112) connected electrically in series to form a Peltier module (110).

7. The device according to claim 6, at least one cell (10) comprising a heat sink means (700, 701, 702, 703) associated with at least one Peltier module (110).

8. The device according to any one of the preceding claims, at least one thermal element being a heating resistor (130) and/or a radiant element (140), for example an infrared (IR) radiant element.

9. The device according to any one of the preceding claims, at least one vibratory element (150) being a piezoelectric element, a ferroelectric element and/or an electromagnetic element.

10. The device according to any one of the preceding claims, at least one thermal and/or vibratory element, preferably all the thermal and/or vibratory elements, being entirely encapsulated in at least one encapsulation layer.

11. The device according to any one of the preceding claims, the at least one encapsulation layer being made of one or more of the following materials: a polymer such as a polyethylene terephthalate (PET), a polyethylene naphthalate (PEN), a polyimide (PI), a polycarbonate (PC) or a silicone; a glass; a metal; or even a non-organic material such as silicon oxide.

12. The device according to any one of the preceding claims, the at least one encapsulation layer being made of a flexible or stretchable material, for example a flexible polymer and/or an elastomer.

13. The device according to any one of the preceding claims, at least one cell (10) further comprising at least one additional heating track (250) under at least one thermal and/or vibratory element, and preferably under at least one bottom conductive track.

14. The device according to any one of the preceding claims, at least one cell (10) having thinned sections (900) between at least two thermal and/or vibratory elements in the thickness of at least one encapsulation layer (501).

15. The device according to any one of the preceding claims, at least one cell (10) comprising a pressure element (800), notably a layer of polymer or of plastic, optionally elastic, disposed over all of said cell and bearing on at least one element, capable of indirectly exerting a pressure against the skin.

16. The device according to claim 1, the Peltier modules (110, 110') having heights greater than the height of the vibratory element (150).

17. The device according to any one of the preceding claims, the matrix (1) comprising multiple cells (10), preferably multiple identical cells.

18. The device according to any one of the preceding claims, at least two thermal and/or vibratory elements being disposed one on top of the other in one and the same cell and/or a matrix of cells.

19. The device according to any one of the preceding claims, at least two thermal and/or vibratory elements being disposed alongside one another in one and the same cell and/or a matrix of cells.
